(19) Europäisches Patentamt
European Patent Office
Office européen des brevets

(11) **EP 2 409 696 B1**

(12) **EUROPEAN PATENT SPECIFICATION**

(45) Date of publication and mention of the grant of the patent:
**27.06.2018 Bulletin 2018/26**

(51) Int Cl.:
*A61K 31/216* (2006.01)     *A23K 20/111* (2016.01)
*A23K 50/40* (2016.01)     *A23L 33/10* (2016.01)
*A23L 33/105* (2016.01)

(21) Application number: **10753295.4**

(22) Date of filing: **17.03.2010**

(86) International application number:
**PCT/JP2010/001902**

(87) International publication number:
**WO 2010/106798 (23.09.2010 Gazette 2010/38)**

(54) **AGENT FOR PROMOTING ENERGY CONSUMPTION**

MITTEL ZUR FÖRDERUNG DES ENERGIEVERBRAUCHS

AGENT DE PROMOTION DE LA CONSOMMATION ÉNERGÉTIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **18.03.2009 JP 2009066623**

(43) Date of publication of application:
**25.01.2012 Bulletin 2012/04**

(73) Proprietor: **Kao Corporation**
**Chuo-ku**
**Tokyo 103-8210 (JP)**

(72) Inventors:
- **MURASE, Takatoshi**
**Haga-gun**
**Tochigi 321-3497 (JP)**
- **MISAWA, Koichi**
**Haga-gun**
**Tochigi 321-3497 (JP)**
- **MINEGISHI, Yoshihiko**
**Haga-gun**
**Tochigi 321-3497 (JP)**
- **OTA, Noriyasu**
**Haga-gun**
**Tochigi 321-3497 (JP)**
- **OHMINAMI, Hideo**
**Haga-gun**
**Tochigi 321-3497 (JP)**

(74) Representative: **Hoffmann Eitle**
**Patent- und Rechtsanwälte PartmbB**
**Arabellastraße 30**
**81925 München (DE)**

(56) References cited:
EP-A1- 1 559 421     EP-A1- 1 716 757
EP-A1- 1 854 780     EP-A1- 1 925 208
WO-A1-00/15215     WO-A1-2007/096643
WO-A1-2008/049194     JP-A- 2003 034 636
JP-A- 2006 045 212     JP-A- 2006 342 145
JP-A- 2008 189 681     JP-A- 2008 189 681
US-A1- 2004 204 338

- Margreet R Olthof ET AL: "Human Nutrition and Metabolism Chlorogenic Acid and Caffeic Acid Are Absorbed in Humans 1", , 1 January 2001 (2001-01-01), XP055258729, Retrieved from the Internet: URL:http://jn.nutrition.org/content/131/1/66.full.pdf [retrieved on 2016-03-16]
- HIROSHI SHIMODA ET AL.: 'Nama Coffee Mame Ekisu no Diet Oyobi Tonyobyo Yobo Sayo' FOOD PROCESSING AND INGREDIENTS vol. 38, no. 11, 2003, pages 54 - 56, XP008167048
- HIDEKATSU TAKEDA ET AL.: 'Ezoukogi Keihisui Ekisu to sono Shuseibun Toyo ga Rat no Yuei Undo ni Okeru Hiro no Hatsugen, Kan-Kin Glycogen Ryo Oyobi Kecchu Shoshihyo ni Oyobosu Eikyo' JOURNAL OF THE MEDICAL SOCIETY OF TOHO UNIVERSITY vol. 37, no. 3, 1991, pages 323 - 333, XP003000010

- **TOSHIRO SAKAI: 'PPARdelta to To-Shishitsu Taisha' SAISHIN IGAKU vol. 62, no. 10, 2007, pages 2380 - 2391, XP008167070**
- **TOSHIRO SAKAI: 'Peroxisome proliferator-activated receptor(PPAR)delta Kasseika ni yoru Metabolic Syndrome Kaizen Sayo' GENOME MEDICINE vol. 8, no. 1, 2008, pages 37 - 47, XP001539082**
- **ANGHEL, S. I. ET AL.: 'Adipose Tissue Integrity as a Prerequisite for Systemic Energy Balance: A CRITICAL ROLE FOR PEROXISOME PROLIFERATOR- ACTIVATED RECEPTOR γ' J. BIOL. CHEM. vol. 282, 2007, pages 29946 - 29957, XP055099057**

**Description**

[Field of the Invention]

The present invention relates to the non-therapeutic use of chlorogenic acids or salts thereof for promoting carbohydrate burning, improving motor functions; inhibiting acetyl-CoA carboxylase 2, and/or inhibiting pyruvate dehydrogenase kinase 4.

[Background of the Invention]

Obesity refers to a condition in which an individual weighs more than usual as a result of excessive accumulation of energy intake from carbohydrate, fat, and the like in the form of fat under the skin or around the viscera. Also, recently, a concept of metabolic syndrome has been the focus of attention. Metabolic syndrome refers to a condition associated with underlying excessive accumulation of visceral fat (obesity), in which one is prone to develop arteriosclerosis complicated with abnormal glucose tolerance (hyperglycemia), high triglyceride (hyperlipidemia), and hypertension, namely, a condition in which one faces an increased risk of developing lifestyle disease.

In Japan, according to the diagnostic criteria for metabolic syndrome published in 2005, males having a waist circumference of 85 cm or more or females having a waist circumference of 90 cm or more who satisfy two or more items of the following three items, which are (1) having blood triglyceride of 150 mg/dl or more or HDL cholesterol of less than 40 mg/dl, (2) having hyperglycemia (fasting blood glucose of 110 mg/dl or more), and (3) having hypertension (130/85 mHg or more), are determined to have metabolic syndrome. Further, according to the investigation conducted by Ministry of Health, Labour and Welfare based on the above diagnostic criteria (May, 2006), 13 million Japanese qualify as having metabolic syndrome, with the number reaching 27 million when those who are likely to develop metabolic syndrome are included.

An increase of people who qualify as suffering from obesity and metabolic syndrome may incur increased medical costs, and obesity and metabolic syndrome are becoming a huge issue not only in Japan but on the worldwide scale.

Because obesity is induced when the amount of energy intake exceeds the amount of energy consumed, in order to ameliorate obesity, a method of decreasing the amount of energy intake from fat, carbohydrate, and the like or a method for increasing the amount of energy consumption by promoting in vivo metabolism by some methods are possible. Therefore, improvements of dietary habit and exercise are considered as an effective method for the prevention and amelioration of obesity and metabolic syndrome.

As for compounds promoting energy consumption, caffeine, chili pepper, and the like which have a sympathetic nerve-activating action are known (Non Patent Documents 1 to 3). Specifically, caffeine in coffee is known to increase heat production (Non Patent Document 4). However, since not only that caffeine is known to have sympathomimetic action and various side effects such as a caffeine intoxication, a diuretic action, and the effect on the fetus but also for its bitterness, it is unsatisfactory in terms of practical utility, such as safety and palatability.

Also, capsiate has recently been discovered as a less pungent capsaicin analog, and its action of promoting energy consumption has been confirmed (Non Patent Document 5).

Meanwhile, although exercise activates energy metabolism mainly in muscle and increases energy consumption, and thus is an effective method for the prevention and amelioration of obesity and metabolic syndrome, performing regular exercise is practically difficult in today's society, which becomes a major cause of the growth of the population of obesity and metabolic syndrome. Accordingly, development of a substance and a method which are safe and available for continuous application and effectively promote energy consumption are demanded.

Also, if the action of exercise can be enhanced, it is considered to serve as effective means for the prevention and amelioration of obesity and metabolic syndrome. Although tea catechin has been reported as an ingredient enhancing the effect of exercise (Patent Document 1), virtually no other ingredients are known.

Further, activation of energy metabolism and promotion of carbohydrate and fat burning are considered to lead to the improvement of motor functions.

Chlorogenic acid (5-caffeoylquinic acid) is a kind of polyphenol contained in coffee and the like, which has been so far reported to have various physiological actions. For example, it is known that chlorogenic acid has a fatty acid synthase-inhibiting action (Non Patent Document 6), and coffee containing chlorogenic acid is known to have a blood glucose absorption-inhibiting action and a body weight-suppressing action (Non Patent Document 7).

Also, it is known that chlorogenic acids have a glucose-6-phosphatase-inhibiting action (Non Patent Document 8), a fat metabolism-activating action by activation of the peroxisome proliferator-activated receptor (PPAR), a preventive and ameliorating action on hyperinsulinemia and hyperleptinemia (Patent Document 2), and a hypotensive action by reduction of oxidative stress (Non Patent Document 9), 3,4-dichlorogenic acid, 3,5-dichlorogenic acid, and 3,4,5-trichlorogenic acid have a maltase-inhibiting action (Non Patent Document 10), and neochlorogenic acid and feruloylquinic acid have a carnitine palmitoyltransferase-activating action (Patent Document 3).

However, the previous findings as noted above are derived from the examination of enzyme activities, inhibitory actions of enzymes, and changes in the blood components, and not associated with energy consumption and the amount of fat or carbohydrate burned.

Coffee is reported to increase heat production as described above, and it is considered that caffeine, which is contained in abundance in coffee, is an active ingredient inducing heat production (Non Patent Document 4).

Therefore, whether or not chlorogenic acids or salts thereof have an action of promoting energy consumption, an action of promoting fat burning, an action of promoting carbohydrate burning, or an action of improving the effect of exercise remains unknown until now.

[Prior Art Documents]

[Patent Document]

[Patent Document 1] U.S. Patent Application Publication No. 2006/0173070
[Patent Document 2] JP-A-2003-34636
[Patent Document 3] JP-A-2006-342145

[Non Patent Document]

[Non Patent Document 1] Dulloo AG., Am. J. Clin. Nutr., 1999, 70: 1040 to 1045.
[Non Patent Document 2] Kawada T., Proc. Soc. Exp. Biol. Med., 1986, 183 (2): 250 to 6.
[Non Patent Document 3] Diepvens K., Am. J. Physiol. Regul. Integr. Comp. Physiol., 2007, 292 (1): R77 to 85
[Non Patent Document 4] Greenberg JA., Am. J. Clin. Nutr., 84, 682 to 693, 2006
[Non Patent Document 5] Ohnuki K., Biosci. Biotechnol. Biochem., 2001, 65 (12): 2735 to 40
[Non Patent Document 6] Li BH., IUBMB. Life, 58 (1), 39 to 46, 2006
[Non Patent Document 7] Thom E., J. Int. Med. Res., 35 (6), 900 to 908, 2007
[Non Patent Document 8] Arion WJ., Arch. Biochem. Biophys., 339 (2), 315 to 322, 1997
[Non Patent Document 9] Suzuki A., J. Hypertens., 24 (6), 1065 to 1073, 2006
[Non Patent Document 10] Matsui T., Biol. Pharm. Bull., 27 (11), 1797 to 1803, 2004

EP 1 854 780 concerns a method of producing a chlorogenic acid composition, which comprises allowing a water-soluble composition extracted from raw coffee beans or roasted coffee beans to be adsorbed to a column filled with an adsorbent and then eluting a chlorogenic acid composition by passing a 0.5 to 20 vol% ethanol aqueous solution.

WO 00/15215 relates to a pharmaceutical composition for treating or preventing an elevated blood lipid level-related disease such as hyperlipidemia, arteriosclerosis, angina pectoris, stroke and hepatic disease in a mammal, which comprises an effective amount of a cinnamic acid derivative of a specific formula.

WO 2007/096643 describes tea containing at least one ester of quinic acid and a trans-cinnamic acid, such as chlorogenic acid, which can aid weight loss.

WO 2008/049194 relates to a composition comprising an extract of green coffee bean, an extract of Salacia oblonga and diacylglycerol for increasing beta-oxidation of fatty acids while maintaining blood glucose concentration through the inhibition of carbohydrate absorption.

JP 2008-189681 concerns a drug or foodstuff for suppressing blood glucose elevation, which comprises chlorogenic acids, preferably at wt. ratios of 120/1 to 1/1 for monocaffeoylquinic acid to dicaffeoylquinic acid.

EP 1 559 421 is directed to a blood fluidity-improving agent, a blood circulation promoter or a cerebrovascular disease-improving agent, containing as an active ingredient thereof one or more ingredients selected from the group consisting of chlorogenic acids, caffeic acids, ferulic acids and pharmaceutically acceptable salts of these acids.

EP 1 925 208 relates to a packaged milk coffee beverage, which has excellent hypertension-alleviating effects and can be ingested routinely. A packaged milk coffee beverage having a pH of from 5 to 7 and satisfying the following conditions (A) to (C) : (A) from 0.01 to 1 wt% of chlorogenic acids, (B) not greater than 0.08 wt% of hydroxyhydroquinone based on a content of said chlorogenic acids, and (C) chlorogenic acids/solid content of coffee ≥ 0. 03 (in terms of weight ratio).

EP 1 716 757 describes a coffee composition having an excellent hypotensive effect, comprising the following components (A) and (B): 0.01 to 1 wt.% of chlorogenic acids (A), less than 0.1 wt.%, based on the weight of the chlorogenic acids, of (B) hydroxyhydroquinone.

[Summary of Invention]

The present invention relates to the following subject matter:

(1) Non-therapeutic use of chlorogenic acids or salts thereof for at least one of the following:

- promoting carbohydrate burning;
- improving motor functions;
- inhibiting acetyl-CoA carboxylase 2; and
- inhibiting pyruvate dehydrogenase kinase 4.

(2) The use according to item (1), wherein the chlorogenic acids are one or more selected from 3-caffeoylquinic acid, 4-caffeoylquinic acid, 5-caffeoylquinic acid, 3,4-dicaffeoylquinic acid, 3,5-dicaffeoylquinic acid, and 4,5-dicaffeoylquinic acid.

(3) The use according to item (2), wherein the chlorogenic acids are one or more selected from 3-caffeoylquinic acid, 4-caffeoylquinic acid, 5-caffeoylquinic acid, and 3,4-dicaffeoylquinic acid.

[Brief Description of Drawings]

[Figure 1] Figure 1 shows graphs illustrating an average amount of energy consumed, an average amount of fat burned, and an average amount of carbohydrate burned during continuous ingestion.
[Figure 2] Figure 2 shows graphs illustrating an average amount of energy consumed and an average amount of fat burned by single ingestion.
[Figure 3] Figure 3 shows graphs illustrating the amount of energy consumed and the amount of fat burned during exercise.
[Figure 4] Figure 4 shows a graph illustrating an inhibitory action of chlorogenic acids on the expression of ACC2 mRNA and PDK4 mRNA.
[Figure 5] Figure 5 shows graphs illustrating inhibitory actions of chlorogenic acids and nine kinds of quinic acid derivatives on the expression of ACC2 mRNA and PDK4 mRNA. In the figure, CQA, FQA, and diCQA represent caffeoylquinic acid, feruloylquinic acid, and dicaffeoylquinic acid, respectively.
[Figure 6] Figure 6 shows a graph illustrating an effect of a packaged beverage containing chlorogenic acids on the promotion of energy consumption.
[Figure 7] Figure 7 shows a graph illustrating the influence of a packaged beverage containing chlorogenic acids on the anaerobic metabolism threshold.

[Embodiments for Carrying out the Invention]

The present invention relates to the non-therapeutic use of chlorogenic acids or salts thereof for at least one of the following:

- promoting carbohydrate burning;
- improving motor functions;
- inhibiting acetyl-CoA carboxylase 2; and
- inhibiting pyruvate dehydrogenase kinase 4.

The present inventors conducted a study on energy consumption, fat burning, carbohydrate burning, and the effect of exercise. As a result, they have found that while chlorogenic acids significantly promote carbohydrate burning, they have inhibitory actions on acetyl-CoA carboxylase 2 (ACC2) and pyruvate dehydrogenase kinase 4 (PDK4), and significantly improve motor functions.
In the present invention, examples of the chlorogenic acids include a compound in which one to two hydroxyl groups selected from the positions 3, 4, and 5 of quinic acid are ester-linked to coffeic acid and/or ferulic acid. Specific examples thereof include 3-caffeoylquinic acid, 4-caffeoylquinic acid, 5-caffeoylquinic acid (chlorogenic acid), 3,4-dicaffeoylquinic acid, 3,5-dicaffeoylquinic acid, and 4,5-dicaffeoylquinic acid, and a mixture of two or more thereof may also be used. Further, feruloylquinic acids such as 3-feruloylquinic acid, 4-feruloylquinic acid, and 5-feruloylquinic acid may be included.

Examples of a salt of chlorogenic acids include a salt with an alkali metal such as sodium and potassium, a salt with an alkaline earth metal such as magnesium and calcium, a salt with an organic amine such as monoethanolamine, diethanolamine, and triethanolamine, and a salt with a basic amino acid such as arginine, lysine, histidine, and ornithine. Also, the chlorogenic acids of the present invention include one containing a free carboxyl group.

The chlorogenic acids or a salt thereof may be used in form of one produced by general chemical synthesis, an extract isolated or purified from nature, or a natural product containing them directly. The extraction may be performed by an ordinary method, and examples of the extract include one derived from coffee beans, apples, grapes, onions, sweet potatoes, and the like. A coffee bean-derived extract is preferable.

Examples of a commercially available preparation containing a large amount of chlorogenic acids or a salt thereof include FLAVOR HOLDER RC-30R (T. Hasegawa. Co., Ltd.), raw coffee bean extract P (Oryza Oil & Fat Chemical Co., Ltd.), and OXCH100 (Toyo Hakko Co., Ltd.). These commercial products can be further purified to increase the purity of chlorogenic acids or a salt thereof. As the production method of chlorogenic acids or a salt thereof, for example, the methods disclosed in JP-A-2006-241006, JP-A-2006-306799, JP-A-2008-94758, JP-A-2008-94759, and the like may be employed.

In general, in vivo energy metabolism is strictly regulated and homeostasis of the amount of energy produced/balance is maintained. Thus, changes in the expression level or the activity of specific molecules are rarely reflected directly in the energy metabolism from an individual-level perspective. Therefore, a macroscale energy state at an individual level cannot be accurately understood only from the results of the expression levels of a specific gene and a specific enzyme activity. The present invention is based on a finding that, as a result of a search for a truly effective ingredient by measuring the amount of energy consumed at an individual level, chlorogenic acids are effective as an agent for promoting carbohydrate burning, and an agent for improving motor functions.

Energy consumption means that nutrients (energy sources) are metabolized in each biological tissue and converted into chemical energy or heat energy. The amount of energy consumed is calculated from the amount of oxygen consumed during that process, and it refers to the amount of physicochemical energy produced on the macroscale at an individual level. Therefore, an action of promoting energy consumption refers to an action of increasing the amount of energy consumed as defined above. Fat burning means that a fatty acid is metabolized in each biological tissue and converted into chemical energy or heat energy.

The amount of fat burned is calculated from the amount of oxygen consumed and the amount of carbon dioxide discharged during the oxidative metabolism process of fat by using, for example, the following equation of Peronnet, et al., and it indicates the amount of fat-derived energy produced at an individual level. Therefore, an action of promoting fat burning refers to an action of increasing the amount of fat burned as defined above.

Carbohydrate burning means that carbohydrate is metabolized in each biological tissue and converted into chemical energy or heat energy. The amount of carbohydrate burned is calculated from the amount of oxygen consumed and the amount of carbon dioxide discharged during that process by using, for example, the following equation of Peronnet, et al., and it indicates the amount of carbohydrate-derived energy produced at an individual level. Therefore, an action of promoting carbohydrate burning refers to an action of increasing the amount of carbohydrate burned as defined above.

$$\text{The amount of fat burned} = 1.695 \times (1 - 1.701 / 1.695 \times \text{respiratory quotient}) \times \text{the amount of oxygen consumed}$$

$$\text{The amount of carbohydrate burned} = (4.585 \times \text{respiratory quotient} - 3.226) \times \text{the amount of oxygen consumed}$$

(with the proviso that the respiratory quotient = the amount of carbon dioxide discharged / the amount of oxygen consumed)

Also, an action of improving motor functions refers to an action of improving physical ability, for example, an aerobic metabolic ability during exercise, which is expressed as an anaerobic metabolism threshold.

In the present invention, specific examples of the action of inhibiting acetyl-CoA carboxylase 2 include inhibitory actions on the expression of ACC2 mRNA and ACC2 protein, and the ACC2 activity. ACC2 is abundantly expressed in muscle and liver, and has an action of converting acetyl CoA into malonyl CoA. From a study of an ACC2 knockout mouse, it has been revealed that inhibition of ACC2 promotes fat and carbohydrate burning to promote energy consumption (Choi CS. et al., Proc. Natl. Acad. Sci. USA, 2007; 104: 16480 to 16485). Also, combined with research findings on an agent for inhibiting ACC (Savage DB et al., J. Clin. Invest. 2006; 116: 817 to 824), etc., it can be said that an agent for inhibiting ACC2 is effective as an agent for promoting energy metabolism.

In the present invention, specific examples of the action of inhibiting pyruvate dehydrogenase kinase 4 (PDK4) include inhibitory actions on the expression of PDK4 mRNA and PDK4 protein, and the PDK4 activity. PDK4 is an enzyme responsible for switching the energy source of a living body by phosphorylation of a pyruvate complex, and inhibition of PDK4 increases the production of acetyl-CoA from glucose by enhancing the activity of a pyruvate dehydrogenase complex responsible for the decarboxylation of pyruvic acid into acetyl-CoA (Sugden, M.C., and Holness, M.J. (2003) Am. J. Physiol. Endocrinol. Metab., 284, E855 to E862). Therefore, inhibition of PDK4 is known to lead to promotion of utilization (oxidation) of carbohydrate.

These actions are fundamentally different from the previously reported fatty acid synthase-inhibiting action (an action of inhibiting fatty acid synthase, which is an enzyme responsible for fatty acid synthesis in liver and adipose tissues, in vitro), blood glucose absorption-inhibiting action (an action of inhibiting the amount of glucose absorbed when coffee containing a chlorogenic acid and sucrose are simultaneously ingested), glucose-6-phosphatase-inhibiting action (an action of inhibiting the activity of glucose-6-phosphatase, which is an enzyme responsible for glyconeogenesis, in vitro), PPAR activation action (an action of promoting PPAR-dependent gene transcription), preventive or ameliorating action on hyperinsulinemia/hyperleptinemia (an action of preventing or ameliorating hyperinsulinemia and hyperleptinemia resulting from insulin and leptin resistances), maltase-inhibiting action (an action of inhibiting maltase, which is maltose hydrolase, in vitro), and carnitine palmitoyltransferase activation action (an action of promoting the enzyme activity of liver carnitine palmitoyltransferase, which is an enzyme producing acylcarnitine from carnitine and acyl-CoA).

As will be shown in the following Examples, chlorogenic acids or salts thereof have an action of promoting energy consumption, an action of promoting fat burning, an action of promoting carbohydrate burning, an action of improving the effect of exercise, an action of improving motor functions, an ACC2-inhibiting action, and a PDK4-inhibiting action. Accordingly, the chlorogenic acids or salts thereof of the present invention can be used for the promotion of carbohydrate burning, the improvement of motor function, the inhibition of ACC2, and the inhibition of PDK4. At this point, the chlorogenic acids or salts thereof can be used singly, or, in addition to these, substances that are acceptable to objects described below into which the chlorogenic acid or a salt thereof is to be mixed, and that include carriers appropriately selected as needed may be used. It is to be noted that this preparation can be produced by an ordinary method depending on an object into which the chlorogenic acid or a salt thereof is to be mixed. The chlorogenic acids or salts thereof can be used mixed, as an active ingredient, into a pharmaceutical product, a quasi-drug, a food, a beverage, and a feed such as a pet food, for the non-therapeutic promotion of carbohydrate burning, the inhibition of ACC2, the inhibition of PDK4, and the improvement of motor functions.

When the chlorogenic acids or salts thereof are used as an active ingredient of a pharmaceutical product, the pharmaceutical product can be administered in any administration form. While examples of the administration form include oral administration, enteral administration, mucosal administration, and injection, oral administration is preferable. Examples of the dosage form of the preparation for oral administration include a tablet, a coated tablet, a capsule, a soft capsule, a granule, a pulvis, a powder, a sustained release preparation, a suspension, an emulsion, a troche, a liquid medicine for internal use, a sugar-coated tablet, a pill, a fine granule, a syrup, an elixir, and a milky liquid. Examples of the dosage form for parenteral administration include intravenous injection, an intramuscular injection medicine, inhalation, an infusion solution, a suppository, an inhalation medicine, a transdermal absorption medicine, an eye drop, a nasal drop, a cream, a gel, a lotion, a patch, a gel, and a paste.

Also, in the preparation, the agent for promoting energy consumption and the like of the present invention may be used singly or in combination with other pharmaceutically acceptable carriers. Examples of the carrier include an excipient, a binder, a disintegrant, a lubricant, a diluent, an osmotic pressure adjuster, a fluidity promoter, an absorption aid, a pH adjuster, an emulsifying agent, an antiseptic, a stabilizer, an antioxidant, a colorant, an ultraviolet ray absorber, a humectant, a thickener, a glazing agent, an activity enhancer, an anti-inflammatory agent, a disinfectant, a corrigent, an odor-masking agent, a filler, a surfactant, a dispersant, a buffer, a preservative, an adhesive agent, a fragrance, and a coating agent.

Specific examples of the aforementioned carrier include a solid carrier such as lactose, kaolin, sucrose, crystalline cellulose, corn starch, talc, agar, pectin, stearic acid, magnesium stearate, lecithin, and sodium chloride, and a liquid carrier such as glycerin, peanut oil, polyvinyl pyrrolidone, olive oil, ethanol, benzyl alcohol, propylene glycol, and water.

Also, an energy consumption-promoting substance such as caffeine may be further mixed into the aforementioned preparation. When caffeine is mixed in, as for a weight ratio of the content of chlorogenic acids or a salt thereof and that of caffeine, a ratio of chlorogenic acids/caffeine is preferably 20/1 to 1/1, more preferably 15/1 to 1/1, and even more preferably 10/1 to 2/1, in terms of chlorogenic acid.

Although the content of the chlorogenic acids or a salt thereof in the aforementioned preparation varies depending on the kind of the preparation, in the case of a preparation for oral administration, it is preferably 1 to 100% by weight, more preferably 5 to 95% by weight, and even more preferably 10 to 70% by weight in terms of chlorogenic acid in the total weight of the preparation.

When the chlorogenic acids or salts thereof are used as an active ingredient of various foods and beverages, in addition to general foods and beverages, based on the concept of the promotion of carbohydrate burning, the inhibition of ACC2, the inhibition of PDK4, and the improvement of motor functions, they are applicable to functional foods and beverages such as cosmetic foods and beverages, foods and beverages for the sick, foods and beverages with nutrient function claims, or foods and beverages for specified health uses, displaying the aforementioned effects as needed.

The form of the food and the beverage may be solid, semi-solid, or liquid. Examples of the food and the beverage include breads, noodles, confectioneries such as cookies, snacks, jellies, milk products, frozen foods, instant foods such as powdered coffee, processed starch products, processed meat products, other processed foods, beverages such as coffee beverages, packaged beverages such as canned coffee beverages, soft drinks, and fruit juice beverages, soups, seasonings, dietary supplements, and raw materials thereof. Also, similarly to the aforementioned preparation for oral administration, the food and the beverage may be in the form of a tablet, a pill, a capsule, a solution, a syrup, a powder, a granule, and the like.

The food and the beverage in the aforementioned form can be prepared by mixing with, in addition to chlorogenic acids or a salt thereof, other food and beverage ingredients, softeners, oil, emulsifiers, antiseptics, fragrances, stabilizers, colorants, antioxidants, thickeners, adhesive agents, dispersants, humectants, and the like, and further, caffeine as described above in an appropriate combination.

Although the content of the chlorogenic acids or a salt thereof in a food and a beverage varies depending on the manner of use thereof, in the form of a beverage, it is normally 0.01 to 5% by weight, preferably 0.05 to 3% by weight, more preferably 0.1 to 2% by weight, and even more preferably 0.1 to 1% by weight in terms of chlorogenic acids. Also, in jellies, the content is normally 0.01 to 1% by weight, preferably 0.02 to 0.5% by weight, and more preferably 0.05 to 0.3% by weight. Also, in coffee beverages, the content is normally 0.01 to 1% by weight, preferably 0.05 to 1% by weight, and more preferably 0.1 to 1% by weight, and even more preferably 0.15 to 1% by weight.

Also, when the chlorogenic acids or salts thereof are used as an active ingredient of a feed, examples thereof include a feed for livestock such as cows, pigs, chickens, sheep, and horses, a feed for small animals such as rabbits, rats, and mice, a feed for fish and shellfish such as tunas, eels, sea breams, yellowtails, and shrimps, and a pet food for dogs, cats, little birds, squirrels, and the like.

It is to be noted that when producing a feed, the agent for promoting energy consumption and the like are used singly or additionally mixed with ordinarily used feed ingredients, for example, meats such as beef, pork, and mutton, protein, grains, rice brans, sake lees, sugars, vegetables, vitamins, and minerals, and further, gelling agents, shape retaining agents, pH adjusters, seasonings, antiseptics, nutrition supplements, and the like which are ordinarily used in a feed, as needed. The above ingredients can be processed by an ordinary method to produce the feed.

No particular limitation is imposed on the dose or the intake amount of the chlorogenic acids or salts thereof when they are used as an active ingredient of pharmaceutical products and functional foods as long as it is an effective amount. Also, although the dose or the intake amount thereof is variable depending on the condition, body weight, sex, age, or another factor of a subject, normally, the daily dose per adult for oral administration is preferably 100 to 3000 mg, more preferably 300 to 2000 mg, and even more preferably 300 to 1000 mg in terms of chlorogenic acids. Also, while the aforementioned preparation can be administered in accordance with any dosing regimen, it is preferably administered once or in several divided doses daily.

[Examples]

Production Example 1

From pulverized roasted coffee beans (2 kg), 1.45 kg of a coffee extract having a solid concentration of approximately 25% was obtained by hot water extraction. The coffee extract thus obtained was concentrated under reduced pressure to a solid concentration of approximately 35%, followed by spray drying at 195°C to give 300 g of a powdered coffee extract. 270 g of the powdered coffee extract thus obtained was dissolved in 9 L of purified water. 9 L of the coffee solution thus obtained was allowed to flow through a 3 L column filled with a synthetic adsorbent (SEPABEADS SP70, the product of Mitsubishi Chemical Corporation). After washing the column by flowing 3 L of purified water, chlorogenic acids were eluted with 33 L of a 0.1% aqueous solution of sodium hydroxide, and then immediately neutralized to weakly acidic using ion-exchange resin (Amberlite 200 CT: Organo Corporation) and collected. The preparation liquid containing chlorogenic acids thus collected was concentrated under reduced pressure to a solid concentration of approximately 10%, followed by spray drying to give a preparation of high-purity chlorogenic acids (40 g).

The content of chlorogenic acids in the preparation of chlorogenic acids thus produced (sum of the content of each of the below-described molecular species) was 77.1%, and the composition was as follows.

3-caffeoylquinic acid (7.7%), 4-caffeoylquinic acid (15.5%), 5-caffeoylquinic acid (31.9%), 3,4-dicaffeoylquinic acid (9.0%), 3,5-dicaffeoylquinic acid (8.9%), 4,5-dicaffeoylquinic acid (5.3%), 3-feruloylquinic acid (7.6%), 4-feruloylquinic acid (5.6%), and 5-feruloylquinic acid (8.5%). It is to be noted that the preparation of chlorogenic acids thus produced did not contain caffeine.

Production Example 2

In 1L of hot water of 98°C, 100 g of raw coffee beans were stirred and extracted for four hours. After cooling, solid-liquid separation was performed, and the extraction liquid thus obtained was concentrated under reduced pressure to a solid concentration of 20% (w/w) at 40°C. Subsequently, the resulting product was spray dried to give a preparation of chlorogenic acids. The amount of chlorogenic acids in the coffee bean extract thus obtained was found to contain 26.3% by weight of caffeoylquinic acid (CQA), 5.1% by weight of feruloylquinic acid (FQA), and 6.65% by weight of dicaffeoylquinic acid (di-CQA).

Production Example 3

The preparation of chlorogenic acids obtained as above was dissolved in ion-exchanged water so that the chlorogenic acids accounted for 1% by weight. After that, 2N hydrochloric acid was added to 165 g of this solution to adjust pH to 1.3, followed by centrifugation for solid-liquid separation to obtain 164 g of an upper layer part. Subsequently, 54 g of the upper layer liquid thus collected was allowed to flow through a column filled with 7.7 ml of a synthetic adsorbent (trade name SEPABEADS SP 207: the product of Mitsubishi Chemical Corporation). Subsequently, 116 g of a 0.1% sodium hydroxide solution was allowed to flow through the column, and the eluate was neutralized to weakly acidic using ion-exchange resin (Amberlite 200 CT: Organo Corporation) and collected. The preparation liquid containing chlorogenic acids thus collected was concentrated under reduced pressure to give a preparation of high-purity chlorogenic acids. The chlorogenic acids-composition thus obtained had a purity of 80% and contained 70% of monocaffeoylquinic acids, 14% of feruloylquinic acids, and 16% of dicaffeoylquinic acids.

Production Example 4: Preparation of nine kinds of chlorogenic acids

From the preparation of chlorogenic acids obtained in Production Example 1, nine kinds of chlorogenic acids were prepared by the medium-pressure column chromatography system (Yamazen: Ultra Pack ODS-A-40D column, UV detector PREP-UV-10V, fraction collector FR 50N, gradient mixer GR200, degassing unit, pump PUMP-600A). The preparation of chlorogenic acids (2 g) was dissolved in 20 ml of a solution A (acetic acid-methanol-water = 1 : 20 : 80), applied subsequently to the column, and eluted at a flow rate of 10 ml/min while applying a gradient ($0 \rightarrow 100\%$) with the solution A from 0 to 100 minutes, and after that with a solution B (methanol) from 100 to 600 minutes. By this operation, 3-caffeoylquinic acid, 3,4-dicaffeoylquinic acid, 3,5-dicaffeoylquinic acid, and 4,5-dicaffeoylquinic acid were obtained as a single compound. A mixture of 4-caffeoylquinic acid, 5-caffeoylquinic acid, 3-feruloylquinic acid, 4-feruloylquinic acid, and 5-feruloylquinic acid was further purified by the preparative HPLC system (LC-908: Japan Analytical Industry Co., Ltd.) using an Inertsil ODS-3 column (GL Science Inc.). As an eluate, trifluoroacetic acid-methanol-water (1 : 300 : 700) was used at a flow rate of 9 ml/min. Elution of chlorogenic acids was monitored by measuring the absorbance at 325 nm. The structure of each compound was confirmed by $^1$H-NMR (JEOL $\alpha$500 NMR spectrometer: JEOL).

Test Example 1: Evaluation of action of promoting energy consumption, action of promoting fat burning, and action of promoting carbohydrate burning during continuous ingestion

C57BL/6J mice (7-week-old, male: Charles River Laboratories Japan, Inc.) were reared by using standard solid feed CE-2 (Oriental Yeast Co., Ltd.) for one week (room temperature: $23 \pm 2°C$, humidity: $55 \pm 10\%$, a light period of 7 am to 7 pm, ad libitum water intake). The mice were then divided into two groups so that the body weight was equalized between the groups. Subsequently, the mice were administered with either a control diet (25% corn oil, 5% lard, 13% sucrose, 20% casein, 28.5% potato starch, 4% cellulose, 3.5% vitamin (trade name: AIN-76 Vitamin Mixture, Oriental Bio Service, Inc.), 1% mineral (trade name: AIN-76 Mineral Mixture, Oriental Bio Service, Inc.)) or a chlorogenic acids-containing test diet (25% vegetable oil, 5% lard, 13% sucrose, 20% casein, 27.5% potato starch, 4% cellulose, 3.5% vitamin, 1% mineral, 1% preparation of chlorogenic acids (described in Production Example 1)) for eight weeks, and then subjected to expiratory gas analysis in the ninth week.

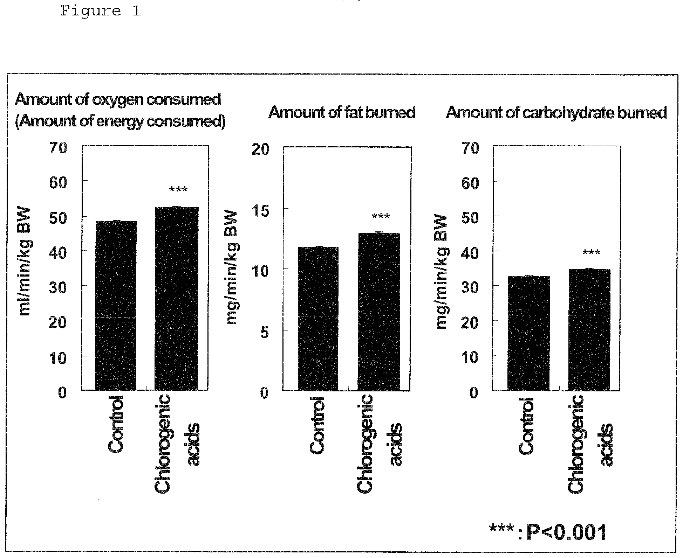

The mice were transferred to an expiratory gas analysis chamber and the expired gas was analyzed for 24 hours with Arco-2000 system (Arcosystem Inc.). In the expiratory gas analysis, the concentrations of oxygen and carbon dioxide in the chamber were measured to calculate the amount of oxygen consumed (the amount of energy consumed) by each mouse as well as the amount of fat burned and the amount of carbohydrate burned by using equation of Peronnet (Peronnet F, and Massicotte D, (1991), Can. J. Sport. Sci. 16: 23 to 29). The results of an average amount of energy consumed, an average amount of fat burned, and an average amount of carbohydrate burned are shown in Figure 1.

In the group of mice that ingested the test diet containing the preparation of chlorogenic acids, the amount of energy consumed, the amount of fat burned, and the amount of carbohydrate burned were found to be significantly increased compared to the control group. Accordingly, chlorogenic acids are effective as an agent for promoting energy consumption, an agent for promoting fat burning, and an agent for promoting carbohydrate burning.

Test Example 2

(Reference Example): Action of promoting energy consumption and action of promoting fat burning by single ingestion

C57BL/6J mice (7-week-old, male: Charles River Laboratories Japan, Inc.) were reared by using standard solid feed CE-2 (Oriental Yeast Co., Ltd.) for one week (room temperature: 23 $\pm$ 2°C, humidity: 55 $\pm$ 10%, a light period of 7 am to 7 pm, ad libitum water intake). The mice were then divided into two groups so that the body weight was equalized between the groups. After eight hours of fasting, under ether anesthesia, the mice were orally administered with either a test aqueous solution containing the preparation of chlorogenic acids produced in Production Example 1 (2% (w/v) total chlorogenic acids) or a control water without chlorogenic acids at 10 ml/kg body weight, followed by oral administration of a sugar/fat mixed emulsion prepared by the following method at 20 ml/kg body weight. The mice were then immediately transferred to an expiratory gas analysis chamber and the expired gas was analyzed for one hour with Arco-2000 system (Arcosystem Inc.). In the expiratory gas analysis, the concentrations of oxygen and carbon dioxide in the chamber were measured to calculate the amount of oxygen consumed (the amount of energy consumed) by each mouse as well as the amount of fat burned by using equation of Peronnet (Peronnet F, and Massicotte D, (1991), Can. J. Sport. Sci. 16: 23 to 29).

<Preparation of sugar/fat mixed emulsion>

Sucrose was dissolved in water to prepare a sucrose solution in advance, to which lecithin (trade name: lecithin, made from eggs, Wako Pure Chemical Industries, Ltd.) and corn oil were added according to the composition shown below. The total volume was brought to 10 ml, and the mixture was emulsified by sonication to give an emulsion.

[Table 1]

| Sucrose | 2 g |
|---|---|
| Corn oil | 2 g |
| Lecithin | 0.08 g |
| DW | an amount to bring the total volume to 10 ml |

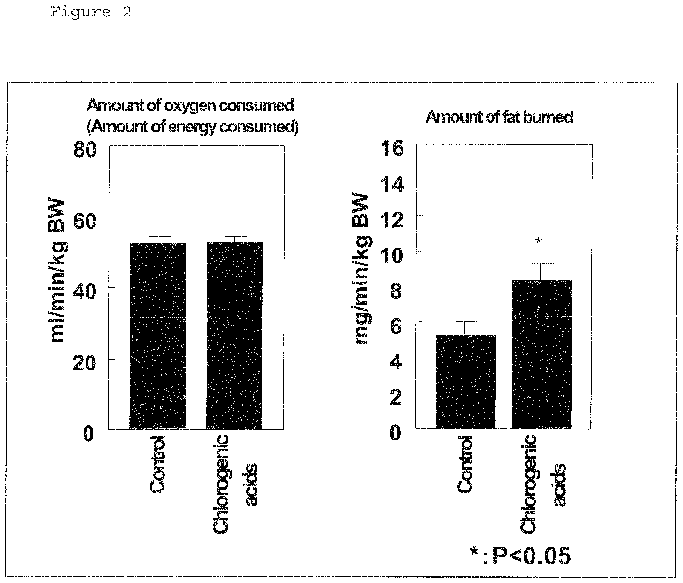

The results of an average amount of energy consumed (amount of oxygen consumed) and an average amount of fat burned are shown in Figure 2.

The amount of fat burned was found to be significantly higher in the chlorogenic acids-administered group. Accordingly, the chlorogenic acids of the present invention are effective as an agent for promoting fat burning. Also, from this experiment, it is understood that chlorogenic acids are effective for promoting dietary fat burning.

Test Example 3: Action of promoting energy consumption, action of promoting fat burning, and action of improving the effect of exercise during exercise

C57BL/6J mice (6-week-old, male: Charles River Laboratories Japan, Inc.) were reared by using standard solid feed CE-2 (Oriental Yeast Co., Ltd.) for one week (room temperature: 23 $\pm$ 2°C, humidity: 55 $\pm$ 10%, a light period of 7 am to 7 pm, ad libitum water intake) to acclimatize them to the environment. At seven weeks old, the mice underwent 5-day training as follows to get familiarized with treadmill running exercise.
Day 1: 5 m/min (10 min) → 10 m/min (10 min) → 15 m/min (10 min)
Day 2: 10 m/min (10 min) → 15 m/min (10 min) → 20 m/min (10 min)
Day 3: 15 m/min (10 min) → 20 m/min (10 min) → 25 m/min (10 min)

Day 4: 15 m/min (10 min) → 20 m/min (10 min) → 25 m/min (10 min)
Day 5: 15 m/min (5 min) → 20 m/min (5 min) → 25 m/min (20 min)

At eight weeks old, the mice were divided into a control diet group (Cont) and a chlorogenic acid group so that the body weight was equalized between the groups. Subsequently, the mice were given either a control diet (5% corn oil, 20% casein, 66.5% potato starch, 4% cellulose, 3.5% vitamin (trade name: AIN-76 Vitamin Mixture, Oriental Bio Service, Inc.), 1% mineral (trade name: AIN-76 Mineral Mixture, Oriental Bio Service, Inc.)) or a test diet containing chlorogenic acids (5% corn oil, 20% casein, 65.5% potato starch, 4% cellulose, 3.5% vitamin, 1% mineral, 1% preparation of chlorogenic acids (described in Production Example 1)) for eight weeks. Also, during this rearing period, the mice underwent running training at 18 m/min (30 min) three times a week.

In the eighth week after initiation of ingestion of the test diet, gas expired during exercise was measured. A measurement test for gas expired during exercise was performed with Arco2000-system (Arcosystem Inc.). The mice were put into treadmill-type chambers for measurement of gas expired during exercise, one mouse per chamber, and kept at rest for two hours to get familiarized with the environment. Subsequently, treadmill was started, and gas expired during exercise was measured for 30 minutes. The treadmill program included 10 m/min (5 min), followed by 15 m/min (5 min), and finally 18 m/min (20 min). A respiratory exchange ratio (RER) was obtained from the amount of oxygen consumed and the amount of carbon dioxide discharged thus measured, and the amount of fat burned was calculated by using equation of Peronnet et al (Peronnet F, and Massicotte D, (1991), Can. J. Sport. Sci. 16: 23 to 29). It is to be noted that when mice refused to run or became incapable of running during the measurement, the measurement was discontinued and the mice were excluded.

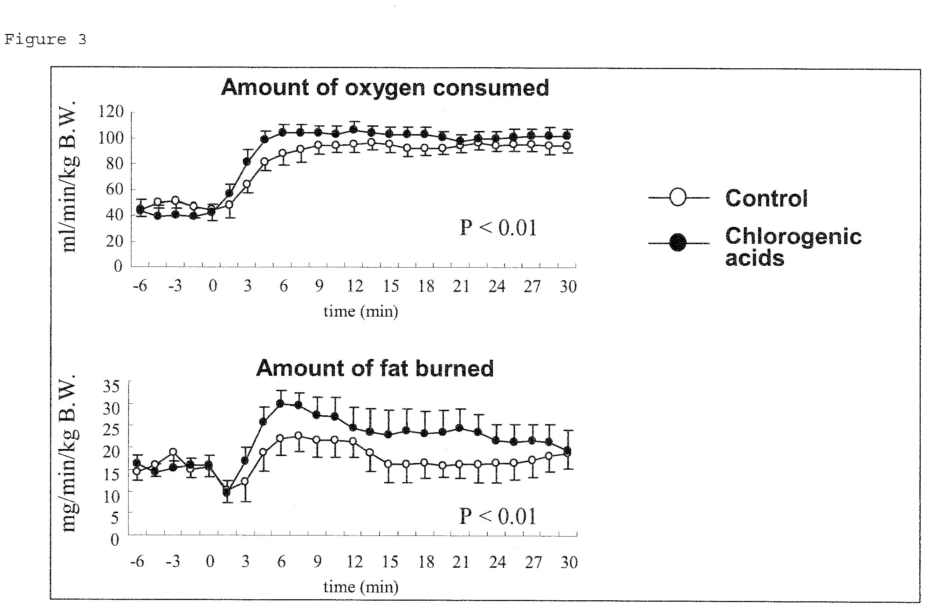

The results of the amount of energy consumed (the amount of oxygen consumed) and the amount of fat burned during exercise are shown in Figure 3. The amount of energy consumed and the amount of fat burned are found to be significantly higher in the group of mice that ingested the test diet containing the chlorogenic acids. Accordingly, the chlorogenic acids of the present invention are effective as an agent for promoting energy metabolism and an agent for promoting fat burning. Also, from this experiment, it is understood that chlorogenic acids are effective for promoting accumulated body fat burning.

Further, while exercise increases energy consumption in general, as understood from the above results, chlorogenic acids further increases the amount of fat burned and the amount of energy consumed when used in combination with exercise. Thus, chlorogenic acids have an action of further enhancing the effect of exercise, and are considered to be effective also as an agent for enhancing the effect of exercise. This further indicates that, when exercise is performed for the prevention or amelioration of obesity and metabolic syndrome, one can achieve the goal more effectively by using the chlorogenic acids in combination.

Test Example 4: Regulatory action of chlorogenic acids on the expression of energy metabolism-related molecule in mouse

C57BL/6J mice (7-week-old, male: Charles River Laboratories Japan, Inc.) were reared by using standard solid feed CE-2 (Oriental Yeast Co., Ltd.) for one week (room temperature: 23 ± 2°C, humidity: 55 ± 10%, a light period of 7 am to 7 pm, ad libitum water intake). The mice were then divided into two groups so that the body weight was equalized between the groups. Subsequently, the mice were given either a control diet (25% corn oil, 5% lard, 13% sucrose, 20% casein, 28.5% potato starch, 4% cellulose, 3.5% vitamin (trade name: AIN-76 Vitamin Mixture, Oriental Bio Service, Inc.), 1% mineral (trade name: AIN-76 Mineral Mixture, Oriental Bio Service, Inc.)) or a test diet containing chlorogenic acids (25% vegetable oil, 5% lard, 13% sucrose, 20% casein, 27.5% potato starch, 4% cellulose, 3.5% vitamin, 1% mineral, and 1% preparation of chlorogenic acids (described in Production Example 1)) for two weeks. The mice were then dissected and the liver was taken. After preparing total RNA according to a standard method, cDNA was prepared using SuperScript first-strand synthesis system (Invitrogen). Quantitative PCR was performed on the cDNA thus obtained by ABI PRISM 7500 Sequence Detection System (Applied Biosystems) using Power SYBR Green Master Mix (Applied Biosystems). Also, the expression level of each gene was corrected using the expression level of 36B4 as an internal standard, and represented as a relative expression level by setting the expression level of control at 100. The primer sequences used for PCR are as shown below.

ACC2-F: ACGAGCACACACAGTCCATG (SEQ ID NO: 1)
ACC2-R: GATGACCTCTGGATGTTCTTG (SEQ ID NO: 2)
36B4-F: CTGATCATCCAGCAGGTGTT (SEQ ID NO: 3)
36B4-R: CCAGGAAGGCCTTGACCTTT (SEQ ID NO: 4)
PDK4-F: AGGGAGGTCGAGCTGTTCTC (SEQ ID NO: 5)
PDK4-R: GGAGTGTTCACTAAGCGGTCA (SEQ ID NO: 6)

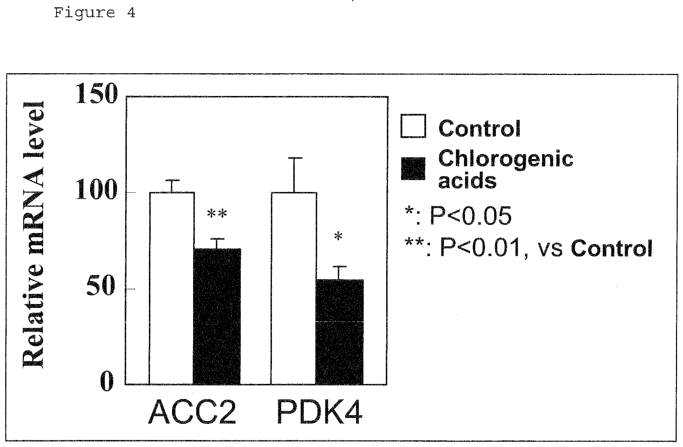

The results are shown in Figure 4. In the liver of mice that ingested chlorogenic acids, the expression of ACC2 mRNA, which negatively regulates energy metabolism, was found to be significantly low. Because inhibition of ACC2 leads to promotion of energy consumption, fat burning, and carbohydrate burning, chlorogenic acids are considered to be effective as an agent for promoting energy metabolism. Also, in mice that ingested chlorogenic acids, the expression of PDK4 mRNA, which negatively regulates carbohydrate metabolism, was found to be significantly low. Thus, it is understood that chlorogenic acids have an action of promoting carbohydrate burning and energy metabolism.

Test Example 5: Regulatory action of chlorogenic acids on the expression of energy metabolism-related molecule in liver cell

A cultured mouse liver cell line (Hepa1-6 cell) was cultured in 10% bovine serum-containing Dulbeco's Modified Eagle's Medium (DMEM) at 37°C, 5% $CO_2$. The cells were seeded in a 6-well plate, and when they reached subconfluency, the medium was replaced by a serum free medium (DMEM, -FBS), followed by culturing for further 12 hours. Subsequently, various samples were added (final concentration: chlorogenic acids (Production Example 1) $2.5 \times 10^{-4}$%(w/v) and each of the chlorogenic acids (Production Example 4) 5 $\mu$M), and after 24 hours of culturing, total RNA was prepared according to a standard method. After preparing cDNA by reverse transcription reaction, quantitative PCR was performed by ABI PRISM 7000 Sequence Detection System (Applied Biosystems) using Power SYBR Green Master Mix (Applied Biosystems). Also, the expression level of each gene was corrected using the expression level of 36B4 as an internal standard, and represented as a relative expression level by setting the expression level of control at 100.

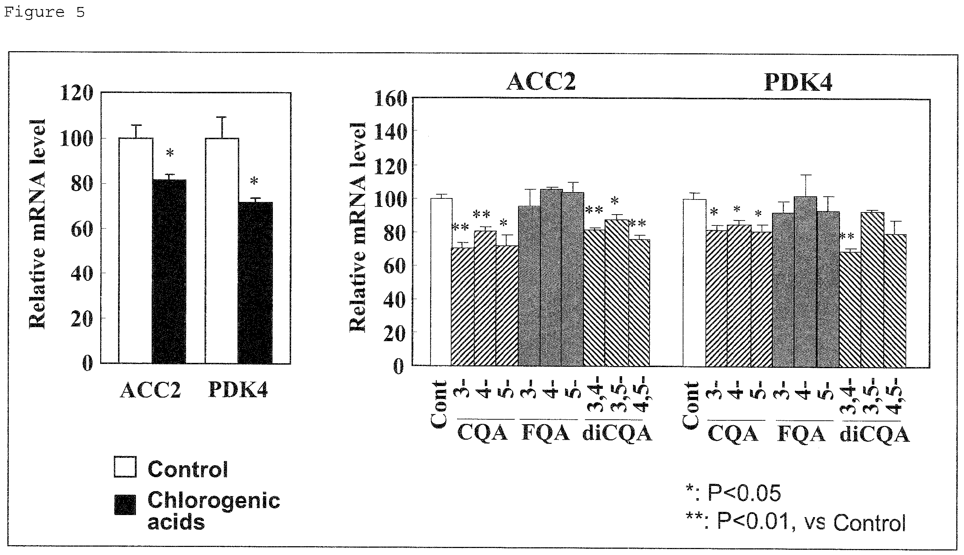

The results are shown in Figure 5. Chlorogenic acids significantly inhibited the expression of ACC2 mRNA, which negatively regulates energy metabolism, and the expression of PDK4 mRNA, which negatively regulates carbohydrate metabolism. Also, as a result of a similar evaluation of nine kinds of chlorogenic acids contained in the preparation of chlorogenic acids (3-caffeoylquinic acid (3-CQA), 4-caffeoylquinic acid (4-CQA), 5-caffeoylquinic acid (5-CQA), 3,4-dicaffeoylquinic acid (3,4-diCQA), 3,5-dicaffeoylquinic acid (3,5-diCQA), 4,5-dicaffeoylquinic acid (4,5-diCQA), 3-feruloylquinic acid (3-FQA), 4-feruloylquinic acid (4-FQA), and 5-feruloylquinic acid (5-FQA)), six kinds of chlorogenic acids of 3-caffeoylquinic acid, 4-caffeoylquinic acid, 5-caffeoylquinic acid, 3,4-dicaffeoylquinic acid, 3,5-dicaffeoylquinic acid, and 4,5-dicaffeoylquinic acid significantly reduced the expression of ACC2 mRNA. Because inhibition of ACC2 leads to promotion of energy consumption, fat burning, and carbohydrate burning, chlorogenic acids, particularly the aforementioned six compounds are considered to be effective as an agent for promoting energy metabolism, an agent for promoting fat burning, and an agent for promoting carbohydrate burning. Also, the expression of PDK4 mRNA was significantly inhibited by 3-caffeoylquinic acid, 4-caffeoylquinic acid, 5-caffeoylquinic acid, and 3,4-dicaffeoylquinic acid. Because inhibition of PDK4 leads to promotion of utilization of carbohydrate, chlorogenic acids, particularly the aforementioned four compounds are considered to be effective as an agent for promoting energy metabolism and an agent for promoting carbohydrate burning.

Test Example 6: Effect of packaged beverage containing chlorogenic acids on the promotion of energy consumption in human (Reference Example)

Using a packaged coffee beverage containing chlorogenic acids as a test beverage and a packaged coffee-like beverage without chlorogenic acids as a control beverage, the effect of chlorogenic acids on the promotion of energy metabolism in humans was verified. This study was performed as a double-blind cross-over study.

- Test beverage (185 ml): chlorogenic acids (3-, 4-, 5-caffeoylquinic acid, total: 306 mg (0.165% by weight)), hydroxyhydroquinone: 0.11 mg, calories: 24 kcal
- Control beverage (185 ml): chlorogenic acids (0 mg), hydroxyhydroquinone: 0.006 mg, calories: 24 kcal

Seven subjects were divided into two groups and drank either one of the above beverages for one week, one bottle daily. After a washout period of one week, each subject drank the other beverage for one week, one bottle daily. After ingesting each beverage for one week, expiratory gas analysis was performed (the amount of oxygen consumed (VO2) and the amount of carbon dioxide discharged (VCO2) were measured) using an expiration analyzer (ARCO2000: Arcosystem Inc.) to evaluate the amount of energy consumed.

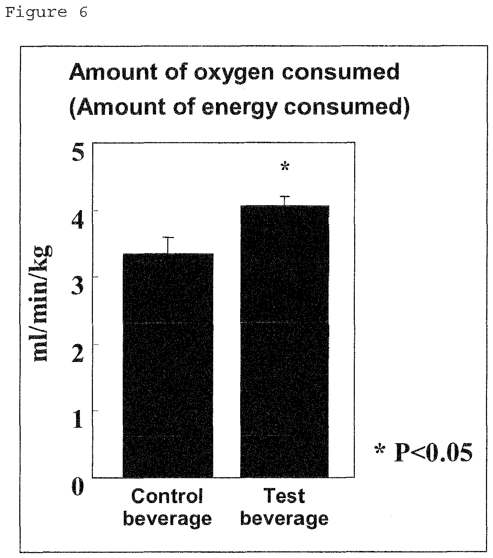

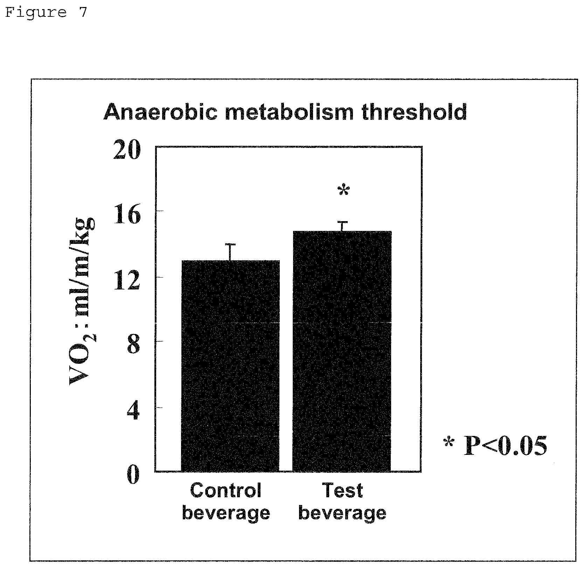

The expiratory gas analysis was performed as follows:
After the subjects were left at rest for 15 minutes and got familiarized with the environment, they ingested test products (either a test beverage or a control beverage) and sandwiches (540 kcal). After 30 minutes, their expiration was analyzed while at rest (for 15 minutes). Thereafter, expired gas was analyzed every 30 minutes, for 15 minutes each time (the results of measurement of an average amount of oxygen consumed (amount of energy consumed) up to two hours are shown in Figure 6). Four hours after ingestion of the test products, the subjects warmed up for three minutes by using an ergometer (20 W, 3 minutes) for exercise, and expired gas during exercise was analyzed while gradually increasing an exercise load up to 60% of the maximal heart rate (maximal heart rate = 220 - age (Achtew, Metabolism, 52: 747 to 752, 2003)) at 15 W/min. The results of anaerobic metabolism threshold (anaerobic threshold: AT) calculated from the results of expiratory gas analysis are shown in Figure 7.

As shown in Figure 6, compared to the control beverage group, the amount of oxygen consumed (energy consumed) in the test beverage group was significantly higher, based on which the effect of the packaged beverage containing chlorogenic acids of the present invention on the promotion of energy consumption was verified.

The anaerobic metabolism threshold refers to an exercise intensity serving as a changing point at which a state in which muscle receives a sufficient oxygen supply shifts to a state in which muscle receives inadequate oxygen, when one is exercising in a way that the exercise intensity is gradually increased. Namely, the anaerobic metabolism threshold refers to the uppermost exercise intensity of aerobic exercise. As shown in Figure 7, as the anaerobic metabolism threshold was significantly high in the test beverage group, it is understood that the aerobic metabolic ability during exercise, namely, the motor function is improved by ingestion of the packaged beverage containing chlorogenic acids of the present invention.

As shown above, the chlorogenic acids of the present invention has an action of promoting energy consumption, an action of promoting fat burning, an action of promoting carbohydrate burning, an action of improving the effect of exercise, an ACC2-inhibiting action, a PDK4-inhibiting action, and an action of improving motor functions.

## Claims

1. Non-therapeutic use of chlorogenic acids or salts thereof for at least one of the following:

    - promoting carbohydrate burning;
    - improving motor functions;
    - inhibiting acetyl-CoA carboxylase 2; and
    - inhibiting pyruvate dehydrogenase kinase 4.

2. The use according to claim 1, wherein the chlorogenic acids are one or more selected from 3-caffeoylquinic acid, 4-caffeoylquinic acid, 5-caffeoylquinic acid, 3,4-dicaffeoylquinic acid, 3,5-dicaffeoylquinic acid, and 4,5-dicaffeoylquinic acid.

3. The use according to claim 2, wherein the chlorogenic acids are one or more selected from 3-caffeoylquinic acid, 4-caffeoylquinic acid, 5-caffeoylquinic acid, and 3,4-dicaffeoylquinic acid.

## Patentansprüche

1. Nicht-therapeutische Verwendung von Chlorogensäure oder Salzen davon für zumindest eine der folgenden:

    Förderung der Kohlenhydratverbrennung,
    Verbesserung der motorischen Funktionen,
    Inhibition von Acetyl-CoA-Carboxylase 2 und
    Inhibition von Pyruvatdehydrogenasekinase 4.

2. Verwendung gemäß Anspruch 1, worin die Chlorogensäuren eine oder mehrere sind, ausgewählt aus 3-Caffeoylchininsäure, 4-Caffeoylchininsäure, 5-Caffeoylchininsäure, 3,4-Dicaffeoylchininsäure, 3,5-Dicaffeoylchininsäure und 4,5-Dicaffeoylchininsäure.

3. Verwendung gemäß Anspruch 2, worin die Chlorogensäuren eine oder mehrere sind, ausgewählt aus 3-Caffeoylchininsäure, 4-Caffeoylchininsäure, 5-Caffeoylchininsäure und 3,4-Dicaffeoylchininsäure.

## Revendications

1. Utilisation non thérapeutique d'acides chlorogéniques ou de sels de ceux-ci pour au moins l'un des éléments suivants :

    - promouvoir la combustion des glucides ;

- améliorer les fonctions motrices ;
- inhiber l'acétyl-CoA carboxylase 2 ; et
- inhiber la pyruvate déshydrogénase kinase 4.

2. Utilisation selon la revendication 1, dans laquelle les acides chlorogéniques sont l'un ou plusieurs sélectionnés parmi l'acide 3-caféoylquinique, l'acide 4-caféoylquinique, l'acide 5-caféoylquinique, l'acide 3,4-dicaféoylquinique, l'acide 3,5-dicaféoylquinique, et l'acide 4,5-dicaféoylquinique.

3. Utilisation selon la revendication 2, dans laquelle les acides chlorogéniques sont l'un ou plusieurs sélectionnés parmi l'acide 3-caféoylquinique, l'acide 4-caféoylquinique, l'acide 5-caféoylquinique, et l'acide 3,4-dicaféoylquinique.

Figure 1

Figure 2

**Amount of oxygen consumed (Amount of energy consumed)**

**Amount of fat burned**

*: P<0.05

Figure 3

Amount of oxygen consumed

Amount of fat burned

Control

Chlorogenic acids

P < 0.01

Figure 4

Figure 5

EP 2 409 696 B1

Figure 6

Amount of oxygen consumed
(Amount of energy consumed)

* P<0.05

Figure 7

**Anaerobic metabolism threshold**

* P<0.05

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20060173070 A
- JP 2003034636 A
- JP 2006342145 A
- EP 1854780 A
- WO 0015215 A
- WO 2007096643 A
- WO 2008049194 A
- JP 2008189681 A
- EP 1559421 A
- EP 1925208 A
- EP 1716757 A
- JP 2006241006 A
- JP 2006306799 A
- JP 2008094758 A
- JP 2008094759 A

### Non-patent literature cited in the description

- **DULLOO AG.** *Am. J. Clin. Nutr.,* 1999, vol. 70, 1040-1045
- **KAWADA T.** *Proc. Soc. Exp. Biol. Med.,* 1986, vol. 183 (2), 250-6
- **DIEPVENS K.** *Am. J. Physiol. Regul. Integr. Comp. Physiol.,* 2007, vol. 292 (1), R77-85
- **GREENBERG JA.** *Am. J. Clin. Nutr.,* 2006, vol. 84, 682-693
- **OHNUKI K.** *Biosci. Biotechnol. Biochem.,* 2001, vol. 65 (12), 2735-40
- **LI BH.** *IUBMB. Life,* 2006, vol. 58 (1), 39-46
- **THOM E.** *J. Int. Med. Res.,* 2007, vol. 35 (6), 900-908
- **ARION WJ.** *Arch. Biochem. Biophys.,* 1997, vol. 339 (2), 315-322
- **SUZUKI A.** *J. Hypertens.,* 2006, vol. 24 (6), 1065-1073
- **MATSUI T.** *Biol. Pharm. Bull.,* 2004, vol. 27 (11), 1797-1803
- **CHOI CS et al.** *Proc. Natl. Acad. Sci. USA,* 2007, vol. 104, 16480-16485
- **SAVAGE DB et al.** *J. Clin. Invest.,* 2006, vol. 116, 817-824
- **SUGDEN, M.C. ; HOLNESS, M.J.** *Am. J. Physiol. Endocrinol. Metab.,* 2003, vol. 284, E855-E862
- **PERONNET F ; MASSICOTTE D.** *Can. J. Sport. Sci.,* 1991, vol. 16, 23-29
- **ACHTEW.** *Metabolism,* 2003, vol. 52, 747-752